# FASCICULE DE BREVET EUROPEEN

(11) **EP 0 742 007 B1**
(45) Date de publication et mention de la délivrance du brevet: **05.11.2003**
(21) Numéro de dépôt: 96440037.8
(22) Date de dépôt: 07.05.1996
(51) Int. Cl.: A61K 7/48

(54) **Utilisation d'extraits de graines de cassia enrichis en galactomannanes dans des produits cosmétiques**
Verwendung von an Galactomannanen angereicherten Cassiasamenextrakten in kosmetischen Zusammensetzungen
Use of cassia seed extracts enriched in galactomannans in cosmetic compositions

(30) Priorité: 12.05.1995 FR 9505815
(43) Date de publication de la demande: 13.11.1996
(73) Titulaire: Cognis France S.A., 31360 Saint Martory (FR)
(72) Inventeur: Pauly, Gilles, 54280 Seichamps (FR); Pauly, Marc, 57170 Château-Salins (FR)
(74) Mandataire: Nuss, Pierre

(56) Documents cités:
- EP-A- 0 121 960
- EP-A- 0 139 913
- WO-A-95/21199
- FR-A- 2 661 833

## Description

La présente invention a notamment pour objet l'utilisation d'extraits de graines de Cassia enrichis en galactomannanes ou dérivés, dans des applications cosmétiques notamment dermatologiques.

Cassia angustifolia Vahl est une légumineuse (Cesalpiniées) originaire d'Inde dont les noms communs sont senné indien, senne Tinnevally, Meca senne.

C'est un sous-arbrisseau herbacé annuel d'environ 0,75m, dont les fruits sont des gousses de 4 à 7 cm de long et de 2 cm de large, fines et plates et qui contiennent 5-7 graines, de couleur brun foncé.

Les graines des plantes du genre Cassia sont riches en polysaccharides et ceux de nombreuses espèces de Cassia ont été isolés et leur structure déterminée.

Les polysaccharides extraits des graines de Cassia sont des galactomannanes.

Ces derniers sont déjà extraits industriellement des graines de Cyamopsis tetragonoloba (Gomme Guar) et de Ceratonia siliqua (Caroube), de Cesalpinia spinosa (Gomme Tara) et de Cassia tora/obtusifolia, et sont constitués par une chaîne principale de mannoses liés en β(1-4) sur lesquels sont substitués des molécules de galactoses ou des galactanes liés en α(1-6).

Les galactomannanes de ces différentes sources se distinguent par la fréquence de substitution des mannoses de la chaîne principale par les galactanes de ces différences structurales peuvent découler des propriétés particulières notamment de solubilité.

Il est déjà connu d'utiliser ces galactomannanes de Cassia et leurs dérivés dans les industries alimentaire, textile, du papier, de l'imprimerie et cosmétique comme agent épaississant ou comme apprêt. Dans l'industrie cosmétique, il est également connu d'utiliser des galactomannanes extraits de caroube et de gomme guar.

On connaît également l'utilisation des mucilages de graines de Cassia absus (galactomannanes et acides polyuroniques) comme pansement gastrique.

La composition en termes d'oses constitutifs des galactomannanes extraits de Cassia est la suivante :

| Composition en % des oses totaux | Galactomannanes de Cassia | Hydrolysat de galactomannanes de Cassia |
|---|---|---|
| Oses acides | 3 | 2 |
| Arabinose | 1 | traces |
| Xylose | 3 | 1 |
| Mannose | 64 | 66 |
| Galactose | 27 | 30 |
| Glucose | 2 | traces |

On connaît par ailleurs par le document EP-A-0 139 913, des substances gélifiantes et épaississantes constituées de mélanges synergiques, d'une part, de galactomannanes de Cassia et, d'autre part, de Carrageenan, d'Agar et/ou de Xanthan.

Ces mélanges synergiques présentent, outre leurs propriétés épaississantes et gélifiantes, également des propriétés suspendantes, émulsifiantes, stabilisantes, lubrifiantes, filmogènes et collantes.

Le document précité indique également l'utilisation de ces mélanges notamment dans des applications pharmaceutiques et cosmétiques, sans faire état toutefois d'autres propriétés particulières dans ce cadre d'application.

Le problème posé à la présente invention consiste à proposer de nouvelles applications utiles en cosmétique pour des extraits de graines de Cassia.

Or, les inventeurs ont constaté de manière inattendue et surprenante que les galactomannanes de graines de Cassia présentaient des propriétés et des effets particuliers supplémentaires les rendant utilisables comme principes actifs en cosmétique.

Ainsi, l'invention a pour objet l'utilisation d'extraits enrichis en galactomannanes de graines de cassia, présentant une composition fractionnaire en oses constitutifs identique à celle existant dans lesdites graines à l'état naturel, en tant qu'agents actifs hydratants et élastifiants dans un produit cosmétique à usage topique pour la peau et/ou les phanères, lesdits extraits étant présents dans le produit cosmétique à usage topique selon une fraction comprise entre 1 % et 25%.

Ces extraits de graines de Cassia pourront être obtenus par les techniques classiques d'extraction et d'isolement des polysaccharides, telle que l'extraction à chaud ou à froid par des solutions aqueuses, hydroalcooliques ou alcooliques, en milieu acide (ou basique), suivie d'une précipitation par un solvant organique. Une étape de réduction des protéines contaminantes par protéolyse enzymatique sera avantageusement incluse dans le cas des graines de cassia.

La matière première sera constituée préférentiellement par les graines de Cassia notamment angustifolia (mais également éventuellement obovata, acutifolia, occidentalis, fistula ou absus) réduites en poudre par broyage.

La farine de Cassia ainsi obtenue, est de couleur jaune et contient entre 15 et 30 % de protéines et entre 2 et 10 %de lipides.

La farine peut être initialement lavée à froid ou à chaud dans un solvant organique (éthanol, méthanol, etc.) ou un mélange hydroalcoolique pour en extraire au maximum les composants colorés ou indésirables (colorant, lipides).

Selon une variante du procédé d'obtention, cette étape de lavage/décoloration peut être réalisée directement en fin de procédé sur la fraction polysaccharidique enrichie en galactomannanes.

Les galactomannanes sont ensuite extraits de ces farines (traitées ou non) par un solvant adapté tel que, par exemple, de l'eau chaude.

L'extraction est réalisée en dispersant la poudre de graines dans une quantité de solvant extractif 20 à 40 fois supérieur en poids à ladite poudre et la température de la solution au cours de l'extraction est maintenue à une valeur comprise entre 20°C et 140°C de préférence entre 80°C et 95°C.

La durée d'extraction des galactomannanes est, dans les conditions de température et d'agitation mentionnées ci-dessus, variable entre 30 minutes et plusieurs heures. L'essentiel de l'extraction est cependant généralement achevé au bout de deux heures.

Après extraction, la fraction de la farine non solubilisée est séparée de l'extrait liquide contenant les galactomannanes par centrifugation.

L'extrait aqueux de Cassia, bien que constituant une fraction enrichie en galactomannanes, présente encore une coloration jaune et sera donc avantageusement traité en vue de sa décoloration.

Les polysaccharides peuvent être précipités à partir du surnageant en versant ce dernier, sous agitation, dans un solvant organique. Le pH de la solution pourra être ajusté avant précipitation à une valeur adaptée à l'utilisation ultérieure des extraits.

Les solvants utilisables sont connus de l'homme de l'art et peuvent être choisis par exemple dans le groupe formé par l'alcool éthylique, le méthanol et l'acétone, le rapport utilisé étant préférentiellement de 0,5 à 1 volume de solvant pour 1 volume de solution à précipiter.

Le précipité fibreux obtenu est ensuite récupéré par filtration, décantation, centrifugation, tamisage ou pressage, et éventuellement décoloré par lavages successifs.

Dans ces conditions, il est possible d'obtenir après séchage et broyage des galactomannanes décolorés.

La mise en oeuvre du procédé d'extraction de Cassia décrit ci-dessus aboutit à un rendement en polysaccharides bruts de Cassia de 10 à 40 % par rapport à la farine de graines de départ, cette fraction contenant encore une certaine proportion de protéines contaminantes.

Selon une variante de réalisation préférentielle du procédé d'extraction, visant à augmenter la teneur en galactomannanes de Cassia de la fraction extraite, la teneur en protéines résiduelles du précipité polysaccharidique est avantageusement fortement diminuée par la mise en oeuvre des méthodes classiques connues d'hydrolyse des protéines.

La solution ainsi traitée est centrifugée et le polysaccharide obtenu par précipitation, comme décrit précédemment.

Le rendement en polysaccharides de cette variante du procédé d'extraction est d'environ 10 à 30 % par rapport à la farine de graines de départ.

L'incorporation des extraits de graines de Cassia enrichis en galactomannanes dans des produits ou des préparations cosmétiques à usage topique, notamment dermatologique, peut être réalisé sous forme de solution ou de suspension aqueuse, de suspension hydropolyolique ou encore à l'état déshydraté, après remise en solution effectuée de manière extemporanée, l'incorporation s'effectuant préférentiellement après redissolution ou dispersion dans un solvant adéquat ou intégration dans une formulation autorisant l'expression des propriétés spécifiques de ces polymères.

Toutefois, en plus des traitements complémentaires prévus ci-dessus ou en variante par rapport à ces derniers, il peut également être prévu d'autres traitements de la solution riche en galactomannanes ou des extraits enrichis en galactomannanes sous forme solide, comprenant des opérations de modification de la forme et/ou de la constitution des galactomannanes extraits telles que par exemple l'hydrolyse et/ou la dépolymérisation chimique ou enzymatique, partielle ou totale, la polymérisation, la réticulation, la condensation et le greffage, la méthylation ou encore la vectorisation, par exemple du type liposomes, nanosphères, nanocapsules, microsphères, microéponges, clathrates, microcapsules ou analogues.

Les dérivés de galactomannanes précedemment décrits peuvent être utilisés soit sous forme liquide, soit sous forme atomisée ou lyophilisée.

Conformément à l'invention, les galactomannanes extraits des graines de cassia sont utilisés sous forme non modifiée ou modifiée, en particulier sous forme d'hydrolysats obtenus par voie enzymatique ou chimique.

Selon l'invention, lesdits extraits de graines de cassia enrichis en galactomannanes sont utilisés sous forme d'une composition intégrée dans le produit ou la préparation cosmétique à usage topique selon une fraction comprise entre 1 % et 25 %.

Lors de la mise en oeuvre d'un produit cosmétique à usage topique obtenu selon l'invention et destiné à une application cutanée il a été constaté une sensation d'hydratation et de douceur, un confort cutané amélioré, notamment lors de l'application sur une peau sensible ou sèche.

Ces effets résultent directement des propriétés filmogènes et réparatrices des galactomannanes extraits des graines de Cassia.

Une amélioration immédiate des qualités tactiles de la peau est obtenue grâce à la formation en surface d'un réseau macromoléculaire fortement hydraté, à caractère hydratant, élastifiant.

Par ailleurs ces extraits semblent également présenter à long terme des activités immunomodulatrices et immunostimulantes. Ainsi, ils trouveront des applications supplémentaires dans le cadre des produits antivieillissement, après-rasages et après-solaires, ainsi que dans le cadre des produits de maquillage traitants et des produits capillaires (shampooings, baumes, lotions).

A titre d'exemples non limitatifs de réalisations pratiques de l'invention, on décrira ci-après différent(e)s produits ou préparations cosmétiques ou pharmaceutiques à usage topique pour la peau ou les phanères, comprenant des extraits de graines de Cassia enrichis en galactomannanes.

### Exemple 1 :

Un produit cosmétique sous forme de crème pour le visage conforme à l'invention pourra, par exemple, présenter une composition pondérale, constituée à partir des fractions A, B et C suivantes, telle qu'indiquée ci-après.

### Fraction A:

- Alcool cétostéarylique (et) ceteareth-20 5,00 %
- Stéarate de glycérol (et) stéarate de PEG-100 3,50 %
- Stéarate de glycérol 2,50 %
- Alcool cétostéarylique 2,00 %
- Huile de paraffine 3,00 %
- Octyldodécanol 5,00 %

### Fraction B :

- Eau qsp 100,00 %
- Conservateurs qs

### Fraction C:

- Galactomannanes de Cassia 5,00 %

Le procédé de préparation et de fabrication de la crème pour le visage ci-dessus consiste essentiellement à chauffer la fraction A et la fraction B à 80° C, à introduire la fraction A dans la fraction B sous agitation (au moyen d'une turbine) en maintenant la température précitée, à refroidir le mélange à 45° C en maintenant l'agitation, à y ajouter la fraction C et enfin à ramener la préparation finale obtenue à température ambiante, sous agitation planétaire.

### Exemple 2 :

Un produit cosmétique sous forme de gel pour le visage conforme à l'invention pourra, par exemple, présenter une composition pondérale, constituée par les fractions A, B, C, D et E, telle qu'indiquée ci-après.

### Fraction A:

- Eau qsp 100,00 %
- Conservateurs qs
- Glycérine 3,00 %
- Copolyol de Dimethicone 2,00 %

### Fraction B :

- Gomme xanthane 0,40 %

### Fraction C :

- Galactomannanes de Cassia 10,00 %

### Fraction D :

- Polyacrylamide (et) C13-14 isoparaffine (et) laureth-7: 4,00 %

### Fraction E:

- Parfum: 0,40 %

Le procédé de préparation et de fabrication du gel pour le visage susvisé consiste essentiellement à préparer la fraction A à 50° C, à disperser la fraction B dans la fraction A sous agitation (au moyen d'une turbine), à laisser refroidir le mélange A + B à température ambiante, à y ajouter successivement la fraction C et la fraction D en réalisant simultanément une homogénéisation jusqu'à obtention d'un gel homogène, à ajouter la fraction E et enfin à homogénéiser et à désaérer la préparation finale obtenue.

### Exemple 3 :

Un produit cosmétique sous forme de lait pour le corps conforme à l'invention pourra, par exemple, présenter une composition pondérale, constituée à partir des fractions A, B, C, D, E et F suivantes, telle qu'indiquée ci-après.

### Fraction A:

- Alcool cétostéarylique (et) ceteareth-20 1,500 %
- Stéarate de glycérol (et) stéarate de PEG-100 1,000 %
- Stéarate de propylène glycol autoémulsionnable 2,000 %
- Huile de paraffine 4,000 %
- Myristate d'isopropyle 2,000 %
- Triglycéride caprylique/caprique 6,000 %

### Fraction B :

- Eau qsp 100,000%
- Propylène glycol 2,000 %
- Conservateurs qs %

### Fraction C :

- Galactomannanes de Cassia 5,000 %

### Fraction D :

- Gomme xanthane 0,400 %
- Conservateurs 0,060%
- Eau 19,540%

### Fraction E :

- Silicate de magnésium et d'aluminium 0,900 %
- Conservateurs 0,054 %
- Eau 17,049 %

### Fraction F :

- Parfum 0,400 %

Le procédé de préparation et de fabrication du lait pour le corps mentionné ci-dessus consiste essentiellement à préparer séparément les fractions D et E, à préparer la fraction B à 80°C, à y ajouter les fractions D et E refroidies à température ambiante et à maintenir le mélange B + D + E à 80° C. Ensuite, il y a lieu de préparer la fraction A à 80° C, d'ajouter sous agitation (au moyen d'une turbine) ladite fraction A dans le mélange B + D + E à 80° C, d'opérer un refroidissement dudit mélange A + B + D + E, d'ajouter la fraction C à ce dernier lorsqu'il est refroidi à environ 45° C à 50° C, ce sous agitation (au moyen d'une turbine). Enfin, il y a lieu d'ajouter la fraction F lorsque le mélange A + B+ C + D + E précité atteint 40° C, puis de refroidir la préparation finale ainsi obtenue jusqu'à température ambiante, ce sous agitation planétaire.

### Exemple 4

Un produit cosmétique sous forme de shampooing traitant conforme à l'invention pourra, par exemple, présenter une composition pondérale, constituée à partir des fractions A, B, C, D et E suivantes, telle qu'indiquée ci-après.

### Fraction A:

- Eau qsp 100,00 %
- Conservateurs qs
- Chlorure d'hydroxypropyltrimonium / hydroxypropyle guar gomme 0,40 %

### Fraction B :

- Sulfate laureth de sodium (et) lauryl polyglucose 25,00 %

### Fraction C:

- Cocamidopropyle bétaïne 8,00 %
- Cocoate de glyceryl PEG 7 0,50 %
- Diméthicone copolyol 1,00 %
- Parfum 0,30 %

### Fraction D :

- Solution aqueuse de citrate de sodium qsp pH = 6

### Fraction E:

- Galactomannanes de Cassia 5,00 %

Le procédé de préparation et de fabrication du shampooing traitant dont la composition est indiquée ci-dessus consiste essentiellement à préparer la fraction A à 80° C sous agitation (au moyen d'une turbine), à refroidir ladite fraction A à température ambiante, à y ajouter successivement les fractions B, C, D et E, à température ambiante et enfin à filtrer le mélange final obtenu avec un filtre de 80 µm.

### Exemple 5

Un produit cosmétique sous forme d'émulsion capillaire conforme à l'invention pourra, par exemple, présenter une composition pondérale, constituée à partir des fractions A, B, C et D suivantes, telle qu'indiquée ci-dessous.

### Fraction A:

- Stéarate de glycérol (et) stéarate de PEG-100 2,50 %
- Alcool oléylique 2,00 %
- Diméthicone 1,00%
- Cyclométhicone 2,00 %

### Fraction B:

- Eau qsp 100,00 %
- Conservateurs qs
- Chlorure d'hydroxypropyltrimonium guar gomme 1,00 %
- Gomme guar 1,00 %

### Fraction C:

- Galactomannanes de Cassia: 5,00%

### Fraction D:

- Solution acide citrique / citrate de sodium: qs pH = 6

Le procédé de préparation et de fabrication de l'émulsion capillaire décrite ci-dessus consiste essentiellement à préparer la fraction A à 75° C, à préparer séparément la fraction B sous agitation (au moyen d'une turbine) à 75° C, jusqu'à obtention d'une constitution homogène et d'une dispersion parfaite des gommes, à ajouter la fraction A dans la fraction B, en maintenant l'agitation et à refroidir le mélange A + B jusqu'à 50° C, en conservant toujours l'agitation. Ensuite, il y a lieu d'ajouter la fraction C au mélange A + B précité à 50° C, d'homogénéiser le mélange A + B + C résultant, de refroidir ce dernier à température ambiante sous agitation planétaire et enfin d'ajuster le pH du mélange précité avec la fraction D.

Bien entendu, l'invention n'est pas limitée au mode de réalisation décrit. Des modifications restent possibles, notamment du point de vue de la constitution des divers éléments ou par substitution d'équivalents techniques, sans sortir pour autant du domaine de protection de l'invention.

## Revendications

1. Utilisation d'extraits enrichis en galactomannanes de graines de cassia, présentant une composition fractionnaire en oses constitutifs identique à celle existant dans lesdites graines à l'état naturel, en tant qu'agents actifs hydratants et élastifiants dans un produit cosmétique à usage topique pour la peau et/ou les phanères, lesdits extraits étant présents dans le produit cosmétique à usage topique selon une fraction pondérale comprise entre 1 % et 25 %.

2. Utilisation selon la revendication 1, **caractérisée en ce que** les galactomannanes extraits des graines de cassia sont utilisés sous forme non modifiée.

3. Utilisation selon la revendication 1, **caractérisée en ce que** les galactomannanes extraits des graines de cassia sont utilisés sous forme d'hydrolysats obtenus par voie enzymatique ou chimique.

## Patentansprüche

1. Verwendung von an Galactomannanen angereicherten Cassiasamenextrakten, die eine fraktionäre Zusammensetzung aus konstitutiven Monosacchariden aufweisen, die mit jener identisch ist, die in den Samen im Naturzustand vorkommt, als aktive hydratisierende und elastifizierende Wirkstoffe in einem Kosmetikprodukt zur topischen Anwendung für die Haut und/oder Anhangsgebilde der Haut, wobei die Extrakte im Kosmetikprodukt zur topischen Anwendung mit einer Gewichtsfraktion zwischen 1 % und 25 % enthalten sind.

2. Verwendung nach Anspruch 1, **dadurch gekennzeichnet, dass** die Galactomannane, die aus den Cassiasamen extrahiert sind, in nicht modifizierter Form verwendet werden.

3. Verwendung nach Anspruch 1, **dadurch gekennzeichnet, dass** die Galactomannane, die aus den Cassiasamen extrahiert sind, in Form von Hydrolysaten verwendet werden, die auf enzymatischem oder chemischem Wege erhalten werden.

## Claims

1. Use of cassia seed extracts enriched in galactomannans, having a fractional composition of constituent oses which is identical to that existing in said seeds in the natural state, as elasticising and moisturising active ingredients in a cosmetic product for topical use for the skin and/or hair, nails, and teeth, said extracts being present in the cosmetic product for topical use in a proportion of between 1% and 25% by weight.

2. Use according to claim 1, **characterised in that** the galactomannans extracted from cassia seeds are used in an unmodified form.

3. Use according to claim 1, **characterised in that** the galactomannans extracted from cassia seeds are used in the form of enzymatically or chemically obtained hydrolysates.
